# DEMANDE DE BREVET EUROPEEN

(11) **EP 2 030 593 A1**
(43) Date de publication de la demande: **04.03.2009**
(21) Numéro de dépôt: 08163097.2
(22) Date de dépôt: 27.08.2008
(51) Int. Cl.: A61F 2/08

(54) **Ensemble d'instrumentation chirurgicale pour reconstruire un ligament croisé postérieur d'un genou**

(30) Priorité: 29.08.2007 FR 0706061
(71) Demandeur: PHUSIS, 38330 Saint Ismier (FR)
(72) Inventeur: Huet, Jacqueline, 38330, SAINT-ISMIER (FR); Pompee, Christian, 38410, SAINT-MARTIN-D'URIAGE (FR)
(74) Mandataire: Myon, Gérard Jean-Pierre

(57) **Abrégé**

Cet ensemble comporte un ancillaire (10) de protection de parties molles postérieures du genou, notamment de l'artère poplitée. Afin de faciliter les gestes du chirurgien pour récupérer un fil (31) d'arrimage d'une greffe de ligament au niveau de la sortie d'un tunnel réalisé à travers le tibia et débouchant sur la face postérieure de l'épiphyse, au niveau d'une zone de cette face recouverte par une extrémité distale (13) d'un corps rigide allongé (12) de l'ancillaire de protection, l'ensemble comporte également un ancillaire (30) de manipulation de ce fil, incluant une partie distale allongée (33, 38), à l'extrémité distale (32) de laquelle est agencée une pièce d'accrochage (40) liée au fil et qui est adaptée, à la fois, pour être introduite, depuis la face antérieure du tibia, dans le tunnel et pour pousser vers l'arrière la pièce d'accrochage jusque dans au moins une ouverture (15) délimitée par l'extrémité distale du corps pour accrocher mécaniquement cette pièce et ce corps l'un à l'autre.

## Description

La présente invention concerne un ensemble d'instrumentation chirurgicale pour reconstruire un ligament croisé postérieur d'un genou.

La reconstruction d'un ligament croisé postérieur de genou est un acte chirurgical délicat car il nécessite de mettre en place une greffe de ligament à l'arrière de l'articulation entre le fémur et le tibia, c'est-à-dire dans les parties molles postérieures du genou qui sont riches en vaisseaux sanguins et en nerfs. En particulier, l'artère poplitée s'étend dans ces parties molles postérieures. Dans ces conditions, une voie d'abord antérieure est privilégiée, avec la contrainte de devoir « franchir » sans l'endommager la zone d'articulation osseuse entre le fémur et le tibia.

Une technique bien établie consiste à réaliser un tunnel à travers le tibia, depuis le côté antérieur de ce dernier jusqu'à la face postérieure de l'épiphyse supérieure du tibia, suivant une direction dirigée à la fois vers l'arrière et vers le haut. De cette façon, une greffe de ligament peut alors être amenée jusque du côté postérieur de l'épiphyse tibiale, en passant par ce tunnel. Toutefois, le tunnel doit être réalisé avec précaution : le foret utilisé pour réaliser le tunnel tibial doit pénétrer suffisamment l'épiphyse pour déboucher sur sa face postérieure, sans pour autant aller au-delà, faute de quoi ce foret endommagerait les parties molles postérieures, avec notamment le risque de perforer l'artère poplitée.

La Demanderesse a donc mis au point et divulgué, par le passé, un ancillaire permettant de protéger les parties molles postérieures du genou lors du forage du tunnel tibial. Cet ancillaire comporte à cet effet un corps rigide allongé, qui, avant le forage, est introduit par l'avant du genou dans l'espace interglénoïdien de l'épiphyse tibiale, jusqu'à ce qu'une extrémité distale de ce corps vienne recouvrir une zone de la face postérieure de l'épiphyse. De cette façon, en prévoyant que le foret débouche sur la zone précitée en fin de forage, le chirurgien est certain que l'extrémité distale du corps de l'ancillaire forme une butée mécanique pour l'extrémité libre du foret, ce qui empêche ce dernière d'atteindre les parties molles situées en arrière de cet extrémité de l'ancillaire.

Avantageusement, cet ancillaire de protection des parties molles postérieures permet également de faciliter la récupération de la greffe de ligament à la sortie du tunnel tibial, c'est-à-dire au niveau du débouché postérieur du tunnel. Pour ce faire, un fil d'arrimage de la greffe, qui présente une certaine rigidité et qui est notamment constitué d'un fil métallique, est introduit en premier dans l'entrée du tunnel, jusqu'à ce que l'extrémité distale de ce fil émerge de la sortie du tunnel. Cette extrémité distale du fil, préalablement conformée globalement en un crochet, met alors en prise une lumière traversante délimitée dans l'extrémité distale du corps de l'ancillaire de protection. En tirant alors doucement cet ancillaire vers l'avant, le chirurgien peut, dans le même mouvement, tracter le fil et, par là, la greffe. En pratique, malgré les précautions prises par le chirurgien, l'extrémité distale du fil se dégage rapidement de l'ancillaire de protection, en particulier avant que cette extrémité du fil ne soit suffisamment amenée vers l'avant, ce qui oblige le chirurgien à utiliser un ancillaire additionnel, tel qu'une petite pince, pour attraper et tracter le fil, en vue de positionner de manière adéquate la greffe à l'arrière du genou.

Le but de la présente invention est d'améliorer l'ensemble d'instrumentation chirurgicale existant, en vue de faciliter les gestes du chirurgien pour récupérer le fil d'arrimage d'une greffe de ligament au niveau de la sortie d'un tunnel tibial débouchant sur la face postérieure de l'épiphyse du tibia.

A cet effet, l'invention a pour objet un ensemble d'instrumentation chirurgicale pour reconstruire un ligament croisé postérieur d'un genou, tel que défini à la revendication 1.

L'idée à la base de l'invention est de réaliser un accrochage mécanique entre l'extrémité distale du fil d'arrimage de la greffe de ligament et l'extrémité distale du corps de l'ancillaire de protection. Grâce à cet accrochage mécanique, le fil peut alors être tracté de manière fiable vers l'avant, par l'ancillaire de protection, sans courir le risque que l'extrémité du fil ne se décroche. Pour ce faire, l'invention repose sur l'utilisation de la pièce d'accrochage liée au fil, qui, sous l'action du chirurgien, peut s'accrocher fermement au corps de l'ancillaire de protection, en passant par la ou les ouvertures prévues à l'extrémité distale de ce corps. L'action du chirurgien est extrêmement aisée, puisqu'elle consiste à solliciter d'avant en arrière l'ancillaire de manipulation ou une partie seulement de cet ancillaire. Ainsi, en un geste rapide et simple à exécuter, le chirurgien accroche fermement le fil d'arrimage au corps de l'ancillaire de protection, par l'intermédiaire de cette pièce d'accrochage. Ce geste est sans danger pour les parties molles postérieures du genou, ces dernières étant protégées par le corps de l'ancillaire de protection, qui empêche que la partie distale de l'ancillaire de manipulation ne puisse être introduite trop profondément dans le tunnel tibial.

En pratique, de nombreuses formes de réalisation différentes peuvent être envisagées, tant en ce qui concerne la partie distale de l'ancillaire de manipulation, qu'en ce qui concerne la pièce d'accrochage. Ainsi, des caractéristiques avantageuses de l'ensemble d'instrumentation défini ci-dessus, prises isolément ou suivant toutes les combinaisons techniquement possibles, sont définies aux revendications dépendantes 2 à 15.

L'invention sera mieux comprise à la lecture de la description qui va suivre, donnée uniquement à titre d'exemple et faite en se référant aux dessins sur lesquels :
- la figure 1 est une vue en perspective d'un ancillaire de protection appartenant à un ensemble d'instrumentation conforme à l'invention, en cours d'utilisation ;
- la figure 2 est une vue schématique en élévation d'une partie de l'ancillaire de protection de la figure 1, associé à un ancillaire de manipulation appartenant à l'ensemble conforme à l'invention, en cours d'utilisation sur une épiphyse tibiale représentée en coupe dans un plan vertical sensiblement médian ;
- la figure 3 est une coupe schématique des ancillaires de la figure 2, dans le plan de coupe de l'épiphyse tibiale considérée à la figure 2, cette épiphyse n'étant pas reprise pour des raisons de visibilité tandis que les ancillaires sont représentés dans une configuration d'utilisation subséquente à celle de la figure 2 ;
- la figure 4 est une coupe partielle selon le plan IV-IV de la figure 3 ;
- la figure 5 est une vue en perspective d'une pièce d'accrochage associée aux ancillaires de la figure 3 ;
- la figure 6 est une vue en élévation, selon la flèche VI indiquée à la figure 3, de l'extrémité distale de l'ancillaire de protection, représentée seule ;
- la figure 7 est une coupe analogue à la figure 3, illustrant les ancillaires dans une configuration d'utilisation subséquente à celle illustrée à la figure 3 ;
- les figures 8 à 11 illustrent un second mode de réalisation d'un ensemble conforme à l'invention, la figure 8 étant une vue analogue à la figure 5 et la figure 9 étant une vue en perspective de l'extrémité distale de l'ancillaire de protection, représentée seule, tandis que les figures 10 et 11 sont des sections schématiques, dans le plan X indiqué à la figure 9, de l'ensemble d'instrumentation en cours d'utilisation, à des moments respectifs analogues à ceux correspondant aux figures 3 et 7 ;
- les figures 12 à 14 illustrent un troisième mode de réalisation d'un ensemble conforme à l'invention, ces figures correspondant à des vues respectivement analogues aux figures 8, 10 et 11 ;
- les figures 15 à 17 illustrent un quatrième mode de réalisation d'un ensemble conforme à l'invention, ces figures correspondant à des vues respectivement analogues aux figures 5, 3 et 7 ;
- les figures 18 à 21 illustrent un cinquième mode de réalisation d'un ensemble conforme à l'invention, ces figures correspondant à des vues respectivement analogues aux figures 8 à 11 ;
- la figure 22 est une vue en perspective illustrant un sixième mode de réalisation d'un ensemble conforme à l'invention, les ancillaires de cet ensemble étant représentés dans une configuration d'utilisation analogue à celle de la figure 2 ;
- la figure 23 est une vue en élévation selon la flèche XXIII de la figure 22, représentant seule l'extrémité distale de l'ancillaire de manipulation ;
- la figure 24 est une vue en élévation selon la flèche XXIV de la figure 23, incluant une coupe de l'extrémité distale de l'ancillaire de protection, dans le même plan de coupe que celui de la figure 3 ;
- la figure 25 est une vue analogue à la figure 24, illustrant les ancillaires dans une configuration d'utilisation subséquente à celle illustrée aux figures 22 à 24 ; et
- la figure 26 est une vue analogue à la figure 22, montrant, à plus grande échelle, les extrémités distales des ancillaires dans une configuration d'utilisation subséquente à celle illustrée à la figure 25.

Dans le présent document, les termes « supérieur », « inférieur », « antérieur », « postérieur », « latéral », « médial », etc. s'entendent dans leur sens anatomique courant, c'est-à-dire qu'ils correspondent à des directions orientées par rapport à un patient se tenant debout à la verticale.

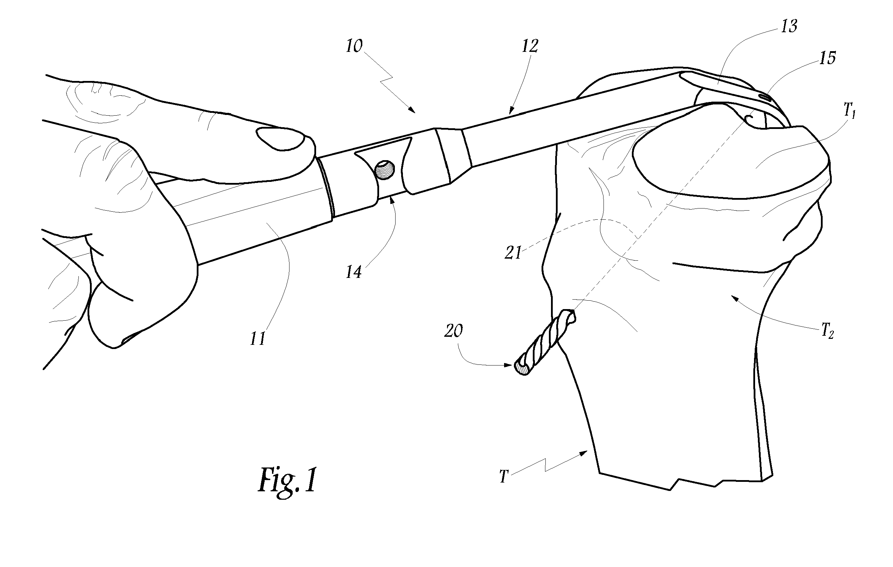

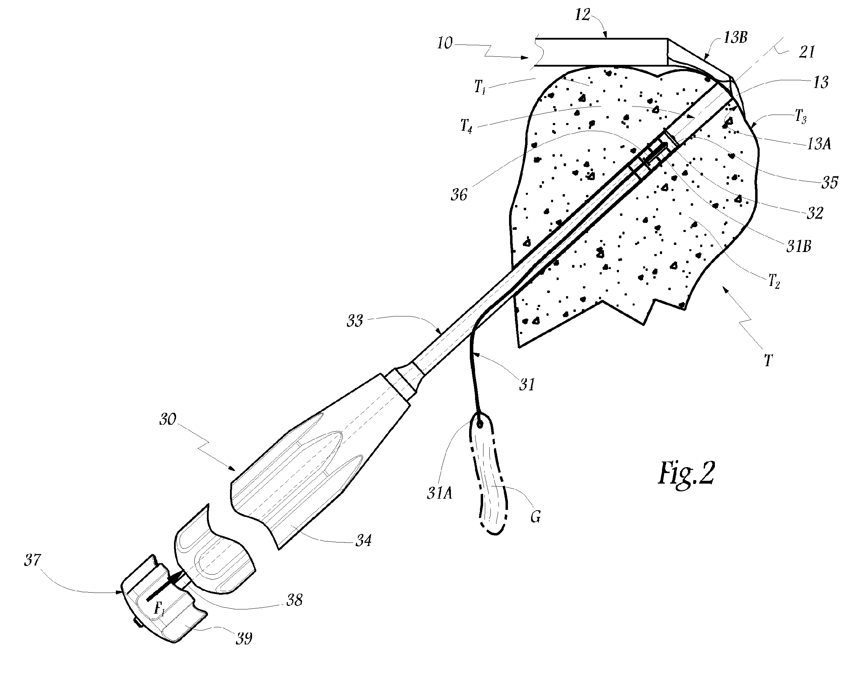

Sur les figures 1 et 2 est ainsi représentée la partie supérieure d'un tibia T d'un genou humain. Pour des raisons de visibilité, le fémur de ce genou n'est pas représenté sur les figures, étant entendu que l'extrémité inférieure de ce fémur s'articule sur la face supérieure de l'extrémité supérieure du tibia, c'est-à-dire sur le plateau tibial T₁ de l'épiphyse supérieure T₂ du tibia.

Ci-après on va décrire un exemple d'intervention chirurgicale visant à reconstruire un ligament croisé postérieur du genou, c'est-à-dire un ligament court et épais qui s'étend de la face postérieure T₃ de l'épiphyse tibiale T₂ à la face latérale du condyle interne du fémur.

Dans un premier temps opératoire, un chirurgien utilise un ancillaire 10, comme représenté sur les figures 1 et 2. L'ancillaire 10 comporte un manche proximal 11 de manipulation manuelle, prolongé à l'opposé du chirurgien par un corps allongé distal 12 rigide, en particulier métallique. Le corps 12 est dimensionné pour être introduit entre le tibia T et le fémur, depuis le côté antérieur du genou. A cet effet, le corps 12 se présente sous la forme globale d'une tige dont l'extrémité distale 13 est conformée en une tête à la fois pointue et incurvée par rapport au reste rectiligne du corps 12. En service, l'extrémité 13 est utilisée pour perforer les tissus mous antérieurs du genou en direction de l'arrière puis passer entre le tibia et le fémur, dans l'espace interglénoïdien délimité entre les cavités glénoïdiennes latérale et médiale du plateau tibial T₁, l'une de ces cavités étant bien visible à la figure 1. L'extrémité 13 est ainsi introduite le long du plateau T₁ jusqu'à recouvrir la face postérieure T₃ de l'épiphyse T₂, au niveau de la zone médiane de cette face postérieure, où débouche vers l'arrière l'espace interglénoïdien. Du côté du fémur, l'extrémité 13 emprunte pour ce faire l'échancrure intercondylienne. L'ancillaire 10 est alors dans sa configuration illustrée aux figures 1 et 2, dans laquelle l'extrémité 13 est incurvée vers le bas par rapport au corps 12, de telle sorte que sa surface inférieure 13A épouse sensiblement la zone médiane de la face postérieure tibiale T₃.

Avantageusement, cette surface 13A présente des bords vifs de manière que l'extrémité 13 peut être utilisée comme rugine pour raboter la face postérieure tibiale T₃ afin d'en détacher des défects osseux, notamment liés au retrait du ligament croisé postérieur d'origine que l'on cherche à reconstruire.

Si besoin, l'ancillaire 10 peut également être alors manipulé par le chirurgien en le tirant vers l'avant suivant une direction sensiblement antéropostérieure, en vue de réduire une éventuelle subluxation postérieure du tibia T par rapport au fémur. Cette réduction est possible grâce à la forme incurvée de l'extrémité 13 qui, en épousant la face postérieure T₃, permet, en quelque sorte, de crocheter l'épiphyse T₂ pour l'entraîner efficacement vers l'avant.

Dans un second temps opératoire, le chirurgien réalise un tunnel T₄ à travers l'épiphyse T₂, au moyen d'un foret de perçage 20, représenté partiellement à la figure 1. Le foret 20 est appliqué depuis la face antérieure de l'épiphyse T₂ et progresse à la fois vers l'arrière et vers le haut suivant un axe rectiligne 21 indiqué en pointillés à la figure 1. Le point d'entrée du foret dans le tibia et la direction de l'axe 21 sont prévus par rapport au tibia de manière que le foret progresse en direction de l'extrémité 13 du corps 12 maintenu immobile par rapport au tibia, jusqu'à ce que, en fin de forage, le foret vienne buter contre la surface 13A. De cette façon, comme visible à la figure 2, le tunnel T₄ ainsi foré est centré sur l'axe 21 et sa sortie débouche sur la surface 13A, étant remarqué que l'extrémité 13 protège efficacement les parties molles postérieures du genou, situées à l'arrière de cette extrémité, dans le sens où le foret ne risque alors pas de pénétrer dans ces parties molles, notamment de perforer l'artère poplitée.

En pratique, pour assurer le bon positionnement relatif du foret 20 et de l'ancillaire 10, le foret est guidé par un canon, non représenté, relié au corps 12 par un arceau de visée, non représenté, agencé à l'avant du genou. A cet effet, l'extrémité proximale du corps 12 présente un relief 14 (figure 1) destiné à coopérer et à fixer l'arceau précité. Avantageusement, l'angle formé entre la direction longitudinale du corps 12 et l'axe 21, dans le plan sensiblement vertical imposé par l'arceau, est réglable, de manière à réaliser le tunnel T₄ dans une zone saine et épaisse de l'épiphyse T₂, tout en garantissant que, en fin de forage, le foret 20 bute contre la surface 13A de l'extrémité 13.

Dans un troisième temps opératoire, après avoir dégagé le foret 20 et, le cas échéant, le canon de guidage de ce foret, le chirurgien utilise un ancillaire 30 représenté sur les figures 2 à 4, 6 et 7, tout en laissant en place l'ancillaire 10 dans sa configuration des figures 1 et 2. En pratique, l'ancillaire 10 est efficacement immobilisé par rapport au tibia T grâce à l'arceau précité, cet arceau étant par exemple maintenu fixement par des broches qui ont été ancrées du côté antérieur de l'épiphyse T₂ lors du deuxième temps opératoire.

L'ancillaire 30 permet au chirurgien de manipuler un fil 31, en vue de faire passer ce fil à travers le tunnel tibial T₄, depuis son entrée jusqu'à sa sortie. Ce fil 31 présente donc une longueur supérieure à celle du tunnel. Une des extrémités longitudinales du fil, référencée 31A à la figure 2, est destinée à être fermement arrimée à une greffe de ligament G. En pratique, pour des raisons précisées plus loin, la greffe G n'est pas reliée au fil 31 lors du troisième temps opératoire, mais à un moment ultérieur, de sorte que, sur la figure 2, la greffe est représentée uniquement en traits mixtes. Cette greffe G présente une forme globalement allongée, dont la section transversale maximale est plus petite que celle du tunnel T₄, de sorte que la greffe est à même d'être glissée dans ce tunnel en étant tractée par le fil 31. En pratique, le fil 31 présente une certaine résistance mécanique, en étant notamment constitué de métal, et son extrémité 31A est destinée à être passée au travers de la greffe G dans une partie tendineuse de cette greffe, pour assurer une bonne liaison mécanique entre la greffe et le fil.

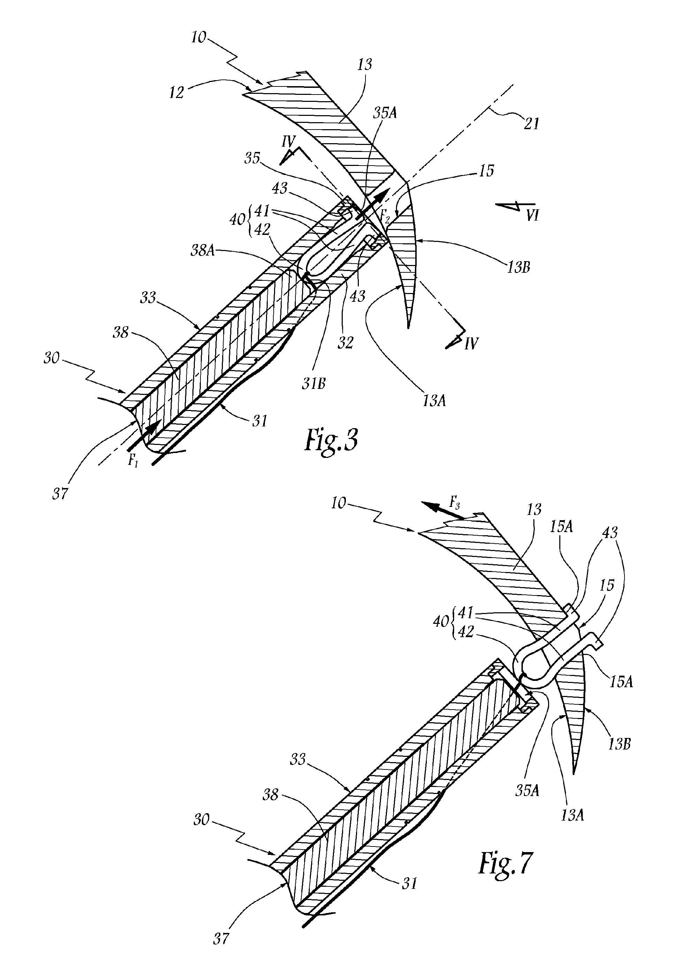

Comme représenté à la figure 2, l'ancillaire 30 est destiné à entraîner dans le tunnel tibial T₄, depuis son entrée jusqu'à sa sortie, l'extrémité 31 B du fil 31 opposée à l'extrémité 31A. Les aménagements concernant l'agencement entre l'extrémité de fil 31 B et cet ancillaire 30 sont représentés plus en détail aux figures 3 à 21, étant entendu que les figures 3 à 7, les figures 8 à 11, les figures 12 à 14, les figures 15 à 17 et les figures 18 à 21 concernent respectivement cinq modes de réalisation possibles pour ces aménagements. Ci-dessous, pour terminer la description de l'intervention chirurgicale visant à reconstruire le ligament croisé postérieur du genou considéré aux figures 1 et 2, on s'intéresse plus en détail au premier mode de réalisation illustré aux figures 3 à 7.

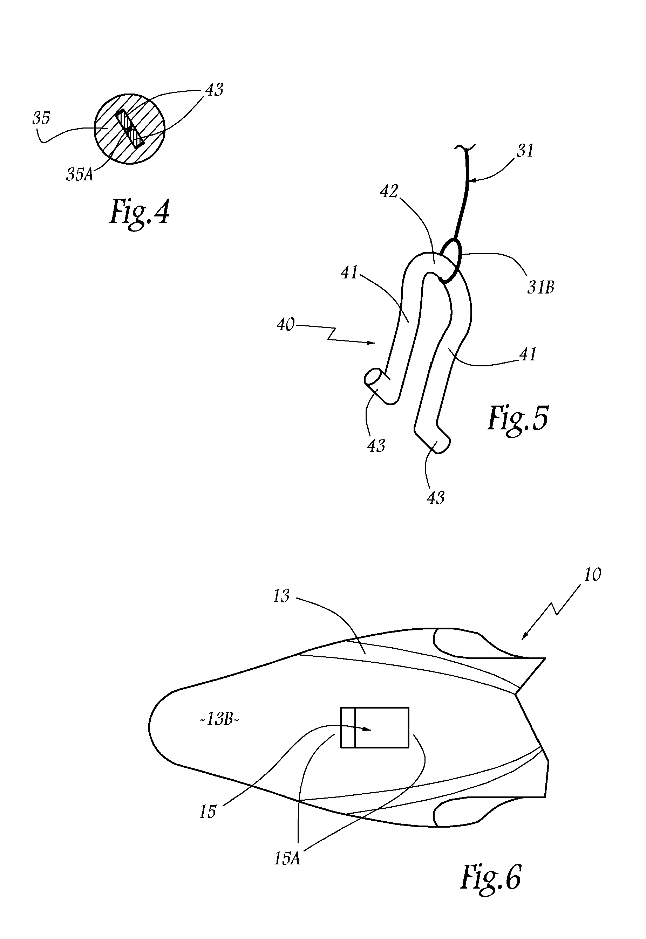

Comme représenté sur les figures 3 à 7, l'extrémité de fil 31 B est liée fermement, par exemple en étant nouée et/ou torsadée, à une pièce 40 en forme de fer à cheval, représentée seule à la figure 5. Cette pièce 40 est adaptée pour s'accrocher à l'extrémité 13 du corps 12 de l'ancillaire 10, sous l'action de l'ancillaire 30. A cet effet, la pièce 40 comporte deux pattes allongées 41 dont des extrémités respectives sont reliées l'une à l'autre par un pont 42, en conférant à la pièce sa forme de fer à cheval, ces pattes 41 et le pont 42 s'étendant globalement dans un même plan. L'extrémité de fil 31 B est avantageusement fixée à la pièce 40 en entourant le pont 42. A l'opposé du pont 42, chaque patte 41 est terminée par une extrémité libre 43 conformée en un bord saillant du reste de la patte, dans la direction opposée à l'autre patte.

Les pattes 41 sont liées au pont 42 de manière élastique, la matière constituant la pièce 40 présentant des propriétés de flexibilité, cette matière étant notamment un acier à ressort. De la sorte, lorsqu'une de ces pattes 41 est écartée d'une position de repos par rapport au pont 42, cette patte tend d'elle-même à être automatiquement rappelée de manière élastique vers sa position de repos.

Au début du troisième temps opératoire, la pièce 40 est portée par l'ancillaire 30. Plus précisément, comme représentée aux figures 3 et 4, cette pièce est logée à l'intérieur de l'extrémité distale 32 d'un manchon 33 rigide, notamment métallique, dont l'extrémité proximale est munie fixement d'un manche de manipulation manuelle 34 (figure 2). Le manchon 33 présente une forme globalement cylindrique, à base circulaire, dont le diamètre est inférieur au diamètre du tunnel tibial T₄. La longueur du manchon 33 étant supérieure à celle du tunnel, on comprend que le chirurgien peut alors introduire l'extrémité distale 32 du manchon 33 dans l'entrée du tunnel T₄ et amener cette extrémité 32 jusqu'à la sortie du tunnel, par manipulation du manche 34. Le manchon 33 est ainsi introduit dans le tunnel T₄, jusqu'à ce que son extrémité 32 butte contre la surface 13A de l'extrémité 13 du corps 12 de l'ancillaire 10, comme représenté aux figures 3 et 4.

A l'intérieur de l'extrémité 32 du manchon 33, la pièce 40 est logée dans une configuration déformée par rapport sa configuration de repos. En effet, en comparaison à leur position de repos, les pattes 41 sont rapprochées l'une de l'autre, dans le plan général de la pièce 40. Pour se faire, l'extrémité 32 est fermée par un bouchon fixe 35 qui délimite une fente traversante 35A. Comme bien visible aux figures 3 et 4, cette fente 35A présente une largeur ajustée sur l'épaisseur de la pièce 40 et une longueur plus petite que la distance séparant les côtés opposés des extrémités 43 des pattes 41 au repos. Ainsi, comme les extrémités 43 des pattes 41 sont logées dans la fente 35A lorsque la pièce 40 est logée dans le manchon, cette fente contraint la pièce dans sa configuration déformée.

Par ailleurs, l'extrémité distale 32 du manchon 33 est ajourée suivant une direction transversale à la direction longitudinale du manchon, pour permettre au fil 31 de traverser la paroi du manchon et atteindre, depuis l'extérieur du manchon, la pièce 40 à laquelle il est lié par son extrémité 31 B. Dans l'exemple de réalisation considéré aux figures, l'extrémité 32 délimite ainsi une ouverture traversante latérale 36 de forme oblongue (figure 2).

Alors que les ancillaires 10 et 30, ainsi que la pièce 40 sont dans la configuration illustrée aux figures 3 et 4, le chirurgien sollicite un poussoir 37 en vue de déplacer la pièce 40 par rapport, à la fois, au corps 12 de l'ancillaire 10 et au manchon 33 de l'ancillaire 30. Comme représenté à la figure 2, ce poussoir 37 est constitué essentiellement par une tige rigide rectiligne 38 reçue à l'intérieur du manchon 33 de manière mobile en translation rectiligne suivant la direction longitudinale de ce manchon. A son extrémité proximale, la tige 38 est munie fixement d'une poignée de manipulation 39 tandis que son extrémité distale 38A ferme vers le bas le logement de réception de la pièce 40 dans l'extrémité distale 32 du manchon. En entraînant le poussoir 37 suivant un mouvement de translation F₁ par rapport au manchon, le chirurgien déplace l'extrémité 38A de la tige vers l'arrière, en direction de la sortie du tunnel T₄. Cette extrémité 38A pousse alors la pièce 40 vers l'arrière, à l'extérieur du manchon 33, en la passant à travers la fente 35A. La pièce 40 s'engage alors à l'intérieur d'une ouverture traversante 15 reliant la surface inférieure 13A à la surface supérieure 13B de l'extrémité 13 du corps 12, comme indiqué par la flèche F₂ à la figure 3. Comme bien visible aux figures 1 et 6, cette ouverture 15 présente la forme d'une fente centrée sur l'axe central 21 du tunnel T₄ et qui s'étend en longueur suivant la direction longitudinale du corps 12. La fente formée par l'ouverture 15 présente une longueur et une largeur sensiblement égales à celles de la fente 35A, de sorte que, sous l'action d'entraînement de l'extrémité 38A de la tige 38, la pièce 40 est poussée successivement à travers la fente 35A et l'ouverture 15 en conservant sa configuration déformée. En pratique, on comprend que cela nécessite que la fente 35A et l'ouverture 15 s'étendent, sur toute leur longueur, en regard l'une de l'autre suivant la direction de la translation F₁. Pour effectuer ce positionnement, le manche 34 porte avantageusement un moyen visuel d'indexation, non représenté, permettant au chirurgien d'indexer la position angulaire de l'ancillaire 30 par rapport à l'ancillaire 10.

L'entraînement en translation du poussoir 37 est poursuivi par le chirurgien jusqu'à ce que les extrémités 43 des pattes 41 émergent à l'extérieur de l'ouverture 15, du côté de la surface 13B, comme représenté sur la figure 7. Les pattes 41 s'écartent alors automatiquement l'une de l'autre, sous l'effet de leur flexibilité par rapport au pont 42, accrochant ainsi mécaniquement la pièce 40 au corps 12. Avantageusement, les extrémités 43 viennent alors en prise avec la surface 13B, en deux zones diamétralement opposées 15A du débouché de l'ouverture 15 sur cette surface. L'accrochage de la pièce 40 s'en trouve renforcé, dans le sens où les bords constitués par les extrémités 43 évitent que la pièce 40 ne repasse par l'ouverture 15 vers l'avant.

Pour passer la pièce 40 à travers l'ouverture 15 jusqu'à l'accrocher au corps 12 de l'ancillaire 10, le poussoir 37 est ainsi translaté de sa position déployée des figures 2 et 3 à sa position escamotée de la figure 7, par rapport au reste de l'ancillaire 30, notamment par rapport au manchon 33. De manière optionnelle non représentée, le poussoir 37 peut être verrouillé dans sa position déployée par des moyens adéquats, que le chirurgien doit retirer ou déverrouiller, afin de lui permettre d'entraîner le poussoir 37 vers sa position escamotée.

Dans un quatrième temps opératoire, le chirurgien dégage l'ancillaire 30, en retirant conjointement le manchon 33 et le poussoir 37 du tunnel T₄, en les passant par l'entrée de ce tunnel. Lors du retrait de l'ancillaire 30, le fil 31 est maintenu à l'intérieur du tunnel T₄, son extrémité 31 B étant fermement immobilisée par rapport au corps 12 de l'ancillaire 10, par la pièce d'accrochage 40, jusqu'à ce que son extrémité 31A passe à travers l'ouverture latérale 36, de l'extérieur à l'intérieur du manchon. Cette extrémité 31A peut alors être arrimée à la greffe G, comme annoncé plus haut.

En tirant ensuite vers l'avant l'ancillaire 10 par son manche 11, comme indiqué par la flèche F₃ à la figure 7, le corps 12 entraîne vers l'avant suivant le même mouvement la pièce 40 accrochée à ce corps et, par là, le fil 31. Le chirurgien peut ainsi tracter de manière fiable la greffe G à l'intérieur du tunnel T₄, en tirant vers lui autant que nécessaire l'ancillaire 10 puis directement le fil 31 lorsque l'extrémité de fil 31 B arrive à disposition du chirurgien, en avant du plateau tibial T₁.

Dans un cinquième temps opératoire, non illustré par les figures, le chirurgien positionne la greffe G de sorte qu'elle s'étende de la sortie du tunnel T₄ à la face latérale du condyle interne du fémur. La greffe G est alors immobilisée dans cette position, au moyen d'une ou de deux vis d'interférence, telles que les vis commercialisées par la Demanderesse sous les noms de « vis habilis » et « tenoscrew », qui mettent en prise la greffe à travers l'épiphyse fémorale et/ou l'épiphyse tibiale.

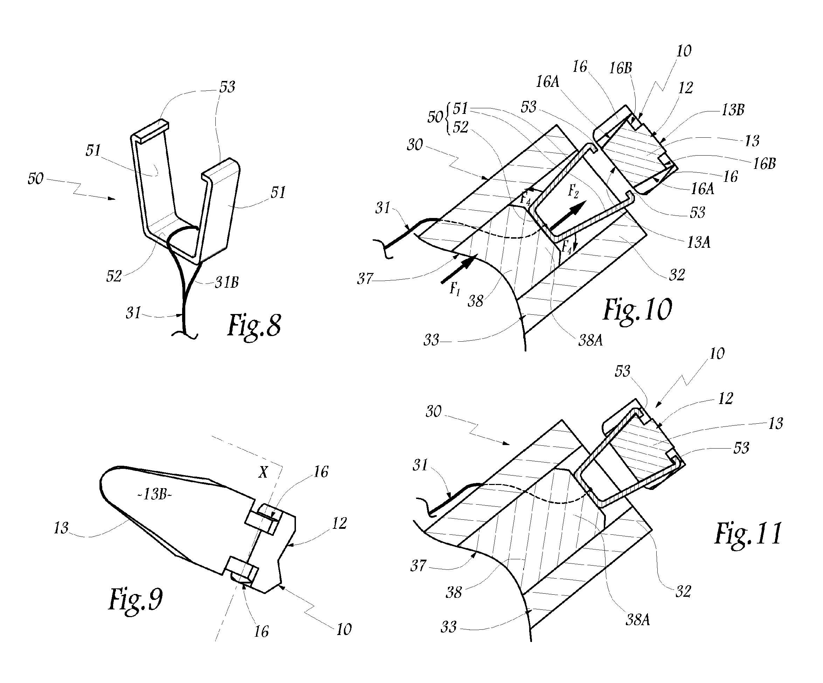

Sur les figures 8 à 11 est illustré un second mode de réalisation de la pièce permettant l'accrochage du fil 31 au corps 12 de l'ancillaire 10. Cette pièce, référencée 50, présente une forme globale en U, qui évoque l'aspect d'une agrafe. Plus précisément, la pièce 50 comprend deux pattes allongées plates 51 dont des extrémités respectives sont reliées l'une à l'autre par un pont allongé plat 52 autour duquel est nouée l'extrémité de fil 31 B. Les deux pattes 51 s'étendent en vis-à-vis l'une de l'autre, en étant reliées au pont 52 de manière élastiquement déformable. Au repos, comme représenté sur la figure 8, les deux pattes 51 s'étendent suivant des directions longitudinales respectives qui divergent l'une de l'autre en s'éloignant du pont 52. L'extrémité libre 53 de chaque patte 51 est conformée en un bord saillant par rapport au reste de la patte, qui s'étend en direction de l'autre patte.

Comme représentée sur les figures 9 et 10, l'extrémité distale 13 du corps 12 n'est pas, comme dans le premier mode de réalisation, traversée par une unique ouverture en forme de fente, délimitée dans la partie médiane de l'extrémité 13, mais est pourvue de deux ouvertures 16, sous forme d'encoches, respectivement délimitées par les flancs, latéral et médial, de l'extrémité 13 et reliant les surfaces inférieure 13A et supérieure 13B. La paroi de fond de chaque ouverture 16 est définie par une surface 16A qui, à son extrémité inférieure, débouche sur la surface 13A et qui, à son extrémité supérieure, débouche sur un épaulement 16B, réalisé dans la surface 13B. Les surfaces 16A des deux ouvertures 16 ne sont pas parallèles l'une à l'autre, mais sont inscrites dans des plans respectifs qui divergent l'un de l'autre en s'éloignant de la surface 13B vers le haut.

L'utilisation de la pièce 50 est analogue à celle de la pièce 40. Ainsi, au début du troisième temps opératoire, la pièce 50 est logée à l'intérieur de l'extrémité distale 32 du manchon 33, de manière à être située en regard de la surface 13A de l'extrémité 13 du corps 12, comme représenté sur la figure 10. Les extrémités 53 émergent partiellement à l'extérieur du manchon, pour faciliter leur coopération ultérieure avec les ouvertures 16, étant entendu que la pièce 50 est, si besoin, retenue dans le manchon par des moyens non représentés dont l'action est interrompue ou vaincue lors de l'entraînement du poussoir 37. Sous l'action du poussoir 37 entraîné suivant la translation F₁, la pièce 50 est progressivement poussée à l'extérieur du manchon 33, en particulier à travers un bouchon fendu, non représenté, analogue au bouchon 35. Les extrémités 53 des pattes, dont l'écartement initial correspond sensiblement à la dimension de la surface 13A prise entre les ouvertures 16, glissent contre les surfaces 16A qui font alors office de rampe pour écarter l'une de l'autre les pattes 51, en augmentant leur divergence relative, par déformation élastique de leur liaison avec le pont 52, comme indiqué par les flèches F₄ à la figure 10. Lorsque les extrémités 53 atteignent le débouché des ouvertures 16 sur la surface 13B, elles pénètrent dans les épaulements 16B, les pattes 31 se rapprochant alors automatiquement l'une de l'autre, sous l'effet de leur flexibilité par rapport au pont 52. Les extrémités 53 sont ainsi en prise avec les épaulements 16B, comme représenté à la figure 11.

Par rapport à la pièce d'accrochage 40 des figures 3 à 7, la pièce d'accrochage 50 présente l'avantage de ne pas être sous contrainte lorsqu'elle est logée dans l'extrémité distale 32 du manchon 33, en attente d'être ultérieurement accrochée au corps 12 de l'ancillaire 10. Cela limite les risques que cette pièce, sous l'action de sa propre élasticité, ne se désengage accidentellement du manchon 33 avant d'être poussée par le poussoir 37 manipulé par le chirurgien. Du fait de l'engagement progressif des extrémités 53 le long des surfaces de rampe 16A, un écart angulaire modéré, typiquement de quelques degrés, entre le manchon et le corps 12 autour de l'axe central du tunnel T₄ peut en outre être corrigé automatiquement par la pièce 50, ce qui facilite le positionnement angulaire relatif des ancillaires 10 et 30.

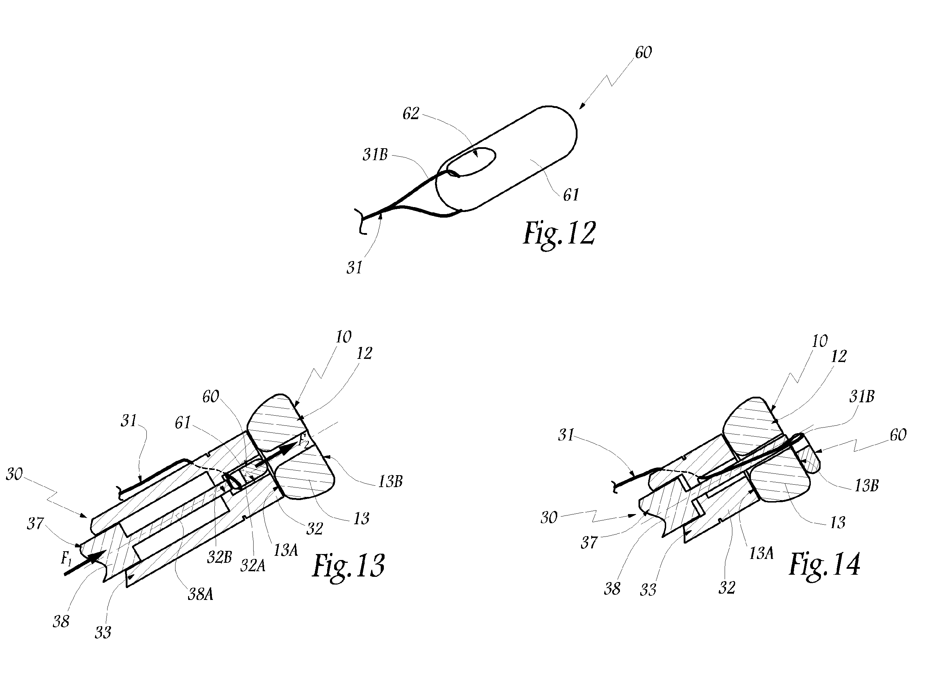

Sur les figures 12 à 14 est illustré un troisième mode de réalisation de la pièce d'accrochage de l'extrémité de fil 31 B au corps 12 de l'ancillaire 10, cette pièce étant référencée 60. A la différence des pièces d'accrochage 40 et 50 qui comportent des parties flexibles par rapport au reste de la pièce, la pièce 60 est constituée d'un corps monobloc allongé 61, de forme globalement cylindrique à base circulaire, dont les extrémités longitudinales sont émoussées. Dans sa partie proximale, ce corps 61 délimite un trou traversant 62 de réception et de fixation de l'extrémité de fil 31 B.

Initialement, c'est-à-dire avant sa sollicitation en vue de s'accrocher à l'ancillaire 10, le corps 61 est logé dans l'extrémité distale 32 du manchon 33 et s'étend en longueur suivant la direction longitudinale du manchon, avec son extrémité distale agencée en regard d'une ouverture traversante cylindrique 17, dont la section transversale est complémentaire, à un jeu près, de celle du corps 61. La pièce 60 est maintenue dans le prolongement rectiligne de l'ouverture 17 par l'extrémité 32 du manchon 33, qui délimite à cet effet un logement 32A de réception du corps 61, sensiblement complémentaire de ce corps.

L'extrémité distale 38A de la tige 38 est conformée en un pion saillant s'étendant de manière rectiligne et centrée par rapport au reste de la tige 38. Lorsque le poussoir 37 est entraîné vers l'avant suivant la translation F₁, ce pion pénètre dans le logement 32A à travers une lumière 32B délimitée dans la paroi de fond du logement, pour pousser la pièce 60 à l'extérieur du logement 32A, comme indiqué par la flèche F₂. Le corps 61 est alors entraîné suivant un mouvement de translation correspondant à travers l'ouverture 17, en étant guidé par la paroi de cette ouverture par coopération des sections transversales complémentaires du corps 61 et de l'ouverture 17, toutes deux sensiblement constantes suivant la direction de translation. La longueur du pion formant l'extrémité 38A de la tige 38 est prévue suffisante pour déplacer ainsi le corps 61, jusqu'à ce que l'extrémité proximale de ce corps atteigne la surface 13B de l'extrémité 13 du corps 12, comme représenté sur la figure 14. Le corps 61 bascule alors de lui-même autour de son extrémité proximale, pour se renverser contre la surface 13B. La dimension longitudinale du corps 61 étant bien supérieure au diamètre de l'ouverture 17, la pièce 60 est alors empêchée de pouvoir se réintroduire d'elle-même dans l'ouverture 17, notamment par traction sur le fil 31. Autrement dit, la pièce 60 est alors accrochée à l'extrémité 13 du corps 12, ce qui permet de tirer l'ancillaire 10 vers l'avant, comme expliqué plus haut.

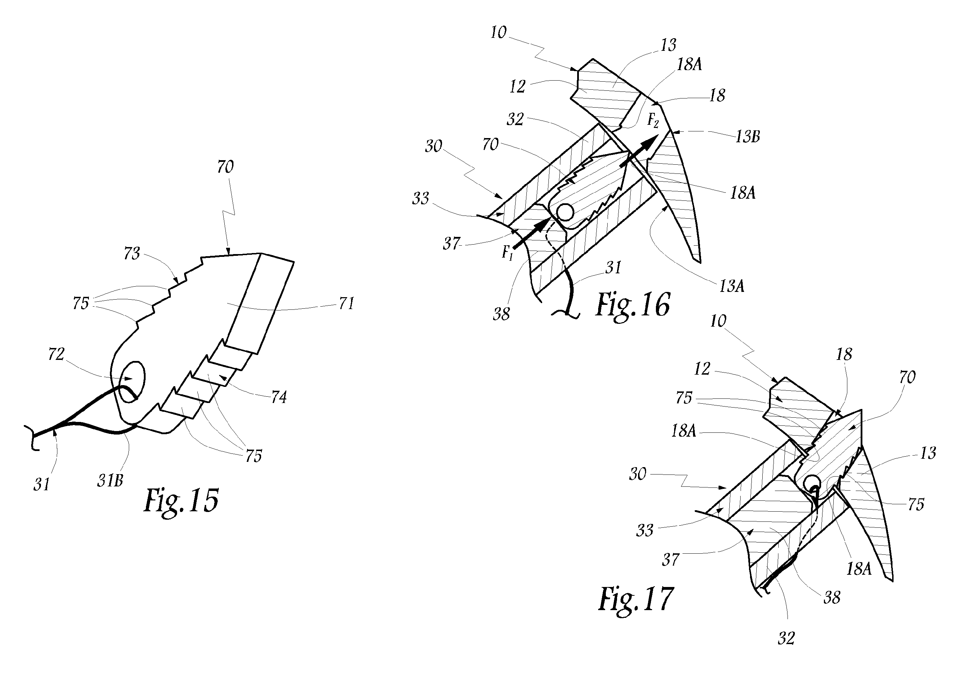

Sur les figures 15 à 17 est illustré un quatrième mode de réalisation de la pièce d'accrochage de l'extrémité de fil 31 B au corps 12 de l'ancillaire 10, cette pièce étant référencée 70. Cette pièce 70 est constituée essentiellement d'un corps monobloc allongé 71 globalement parallélépipédique, muni à son extrémité proximale d'un trou traversant 72 de réception et de fixation de l'extrémité de fil 31 B. Deux de ses flancs opposés 73 et 74 sont respectivement munis de crans saillants 75 répartis sur ces flancs suivant la direction longitudinale du corps 71. L'extrémité 13 du corps 12 délimite une ouverture traversante 18 à travers laquelle le corps 71 est à introduire à des fins d'accrochage. Cette ouverture 18 présente une géométrie sensiblement analogue à l'ouverture 15, à la différence que son débouché sur la surface 13A est muni de deux lamelles 18A disposées en regard l'une de l'autre. Chaque lamelle 18A s'étend en saillie à l'intérieur de l'ouverture 18 depuis la paroi de cette ouverture de sorte que, lorsque la pièce 70 est poussée à travers l'ouverture 18 sous l'action du poussoir 37, comme indiqué par la flèche F₂, les lamelles 18A sont pressées vers l'arrière par les crans 75. Dans ces conditions, chaque cran 75 déforme successivement la lamelle correspondante, par effet de rampe, afin de franchir cette lamelle. Une fois que ce cran a dépassé la lamelle, cette dernière retrouve sa configuration initiale non déformée, par retour élastique de sa structure, empêchant alors par effet de coin le déplacement du corps 71 en sens inverse. Ainsi, les crans 75 forment des crans anti-retour de la pièce 70, accrochant ainsi fermement cette dernière à l'extrémité 13 du corps 12, comme représenté sur la figure 17.

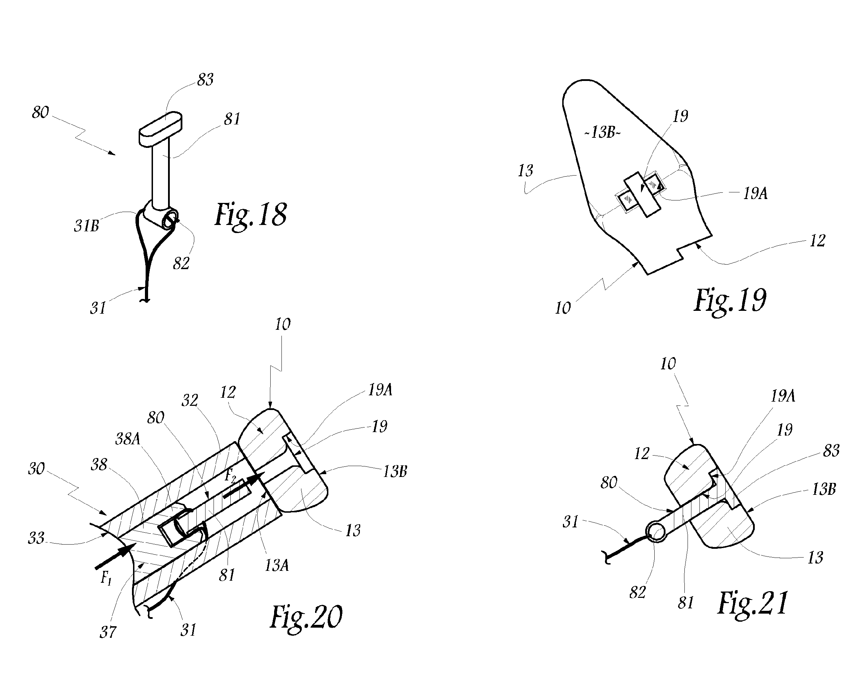

Sur les figures 18 à 21 est illustré un cinquième mode de réalisation d'une pièce d'accrochage de l'extrémité de fil 31 B au corps 12 de l'ancillaire 10, cette pièce étant référencée 80. Comme bien visible à la figure 18, la pièce 80 comporte un corps cylindrique à base circulaire 81 dont l'extrémité proximale délimite un trou traversant 82 de réception et de fixation de l'extrémité de fil 31 B, tandis que son extrémité distale est munie d'une barrette 83 s'étendant en longueur à la perpendiculaire du corps 81, de part et d'autre de ce corps, et ce avantageusement dans des proportions analogues. La largeur de la barrette 83 est sensiblement égale au diamètre du corps 81.

Pour permettre de passer la barrette 83 à travers l'extrémité 13 du corps 12, cette extrémité délimite une ouverture traversante 19 en forme de fente dont la section transversale présente des dimensions égales à la longueur et à la largeur de la barrette 83. La dimension de l'ouverture 19, prise entre les surfaces 13A et 13B, est inférieure à la longueur du corps 81, de sorte que, sous l'action de poussée translative F₁ du poussoir 37, la pièce 81 peut être introduite, depuis l'extrémité distale 32 du manchon 33, à travers l'ouverture 19, jusqu'à ce que la barrette 83 s'étende à l'aplomb de la surface 13B. Par coopération entre l'extrémité proximale du corps 81 et l'extrémité distale 38A de la tige 38, qui délimite un logement de réception complémentaire, la mise en rotation sur lui-même du poussoir 37 permet alors d'entraîner la pièce 80 en rotation sur elle-même autour de l'axe longitudinal du corps 81, et ce sur un quart de tour environ pour que la barrette 83 s'étende en longueur suivant une direction sensiblement perpendiculaire à la direction longitudinale du débouché 19A de l'ouverture 19 sur la surface 13B. Avantageusement, comme représenté à la figure 19, ce débouché 19A est dimensionné pour recevoir de manière sensiblement complémentaire la barrette 83, cette barrette y étant introduite par un mouvement de translation vers l'avant de la pièce 80, soit sous l'effet de son propre poids, soit sous l'effet d'une traction correspondante sur le fil 31. La barrette 83 se trouve alors bloquée dans le débouché 19A, comme représenté à la figure 21, de sorte que la pièce 80 est fermement accrochée à l'extrémité 13 du corps 12.

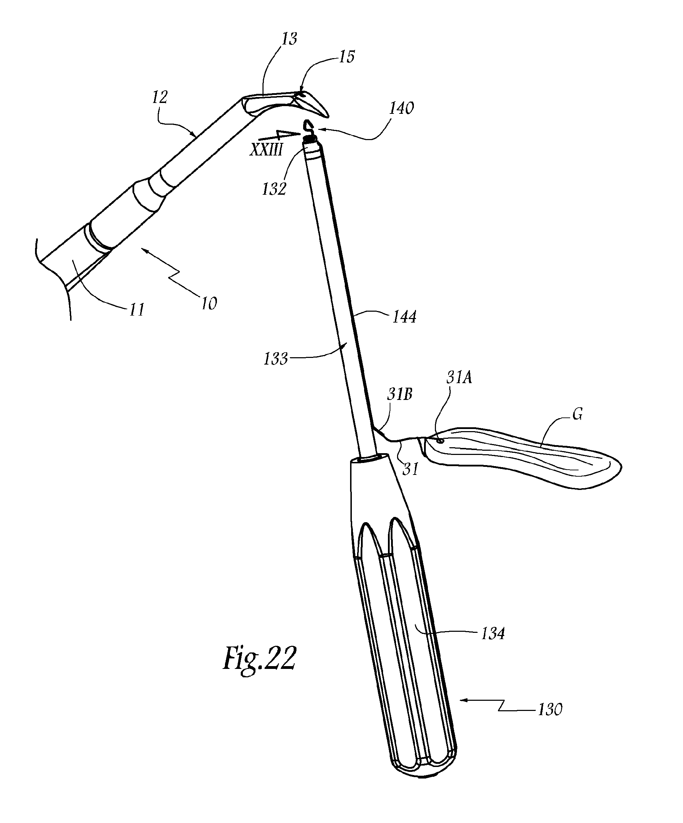

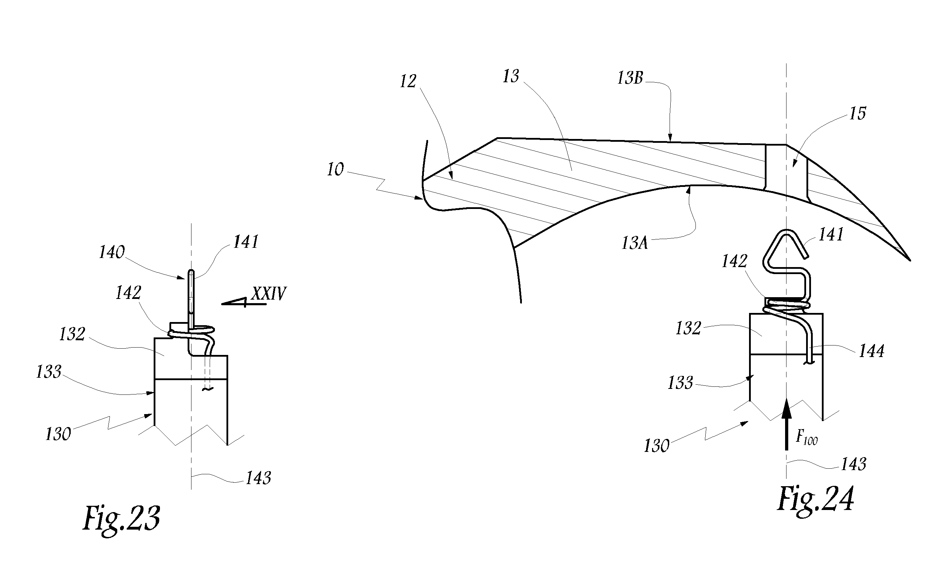

Sur les figures 22 à 26 est illustré un sixième mode de réalisation d'une pièce permettant l'accrochage du fil 31 au corps 12 de l'ancillaire 10, cette pièce étant référencée 140. Ce mode de réalisation induit des aménagements spécifiques de l'ancillaire de manipulation, qui s'en trouve référencé 130. Plus précisément, comme bien visible sur les figures 23 et 24, la pièce 140 présente une structure filaire élaborée qui, comme expliqué ci-après, possède des propriétés élastiques. A cette fin, la pièce 140 est notamment réalisée en un fil d'acier à ressort. Au niveau de son extrémité libre visible sur les figures 23 et 24, cette structure filaire est conformée en une pointe en V 141, globalement inscrite dans un plan géométrique, qui correspond au plan de représentation de la figure 24. Cette pointe en V 141 est convergente en direction opposée au reste de la pièce 140 et se prolonge, de son côté divergent, par un enroulement 142 de la structure filaire précitée sur elle-même, centré sur un axe géométrique 143 sur lequel la pointe en V 141 est également centrée. Cet enroulement 142 s'étend ainsi sur environ un tour autour de l'axe 143, de part et d'autre du plan géométrique contenant la pointe en V 141.

La pièce 140 inclut également une partie filaire rectiligne 144 qui s'étend parallèlement à l'axe 143, depuis l'enroulement 142 en direction opposée à la pointe en V 141. A son extrémité opposée à l'enroulement 142, cette partie rectiligne 144 est liée fermement à l'extrémité 31 B du fil 31 d'arrimage de la greffe G, comme représenté sur la figure 22.

Dans la configuration d'utilisation illustrée sur les figures 22 à 24, la pièce 140 est portée par l'ancillaire 130: l'enroulement 142 coopère mécaniquement avec l'extrémité distale 132 d'une tige rigide 133 de l'ancillaire 130, en étant engagé sensiblement coaxialement autour de cette extrémité, de manière à relier fixement la pièce 140 et la tige 133. Pour ce faire, l'extrémité 132 de la tige 133 délimite une gorge circonférentielle, complémentaire de l'enroulement 142 et interrompue par un méplat diamétral. Lorsque l'enroulement 142 est ainsi fixé à l'extrémité 132 de la tige 133, la pointe en V 141 se trouve disposée en saillie, vers l'arrière, de cette extrémité 132, et ce dans un plan géométrique correspondant au méplat diamétral de cette extrémité, tandis que, comme visible sur la figure 22, la partie rectiligne 144 court le long de la tige 133, avantageusement jusqu'à l'extrémité proximale de cette tige, qui est munie d'un manche de manipulation manuelle 134.

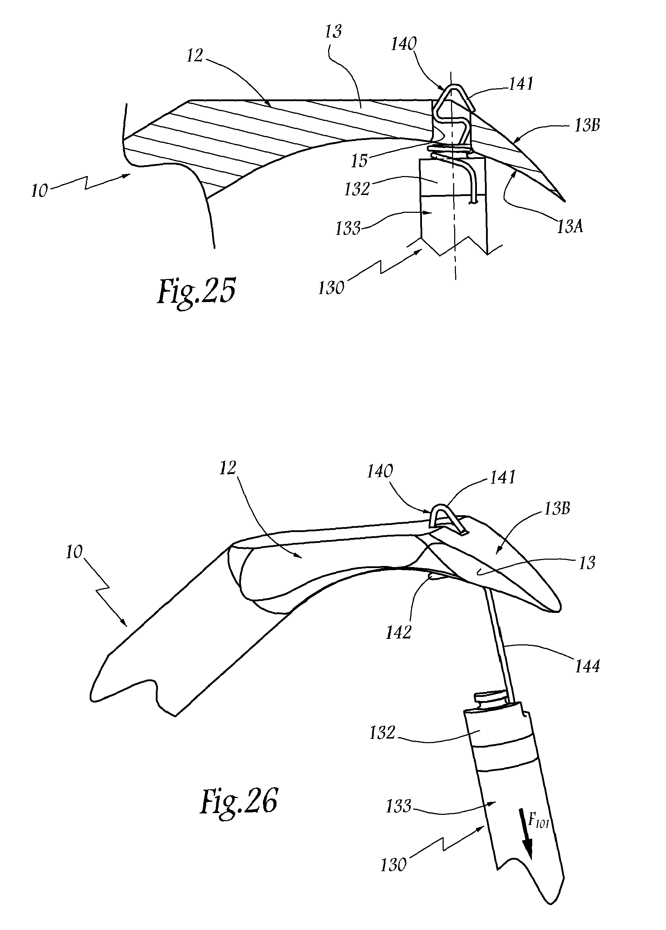

L'ancillaire 130 et la pièce d'accrochage 140 s'utilisent au cours d'une intervention chirurgicale dont les premier et deuxième temps opératoires sont identiques à ceux décrits plus haut en regard de la figure 1. Dans un troisième temps opératoire, analogue au troisième temps opératoire décrit plus haut, le chirurgien introduit l'extrémité distale 132 de la tige 133 dans l'entrée du tunnel tibial T₄ et amène cette extrémité 132 jusqu'à la sortie du tunnel, par manipulation du manche 134, tandis que l'ancillaire 10 est laissé en place dans sa configuration des figures 1 et 2. Lorsque l'extrémité 132 de la tige 133 est à proximité de l'extrémité 13 du corps 12, les ancillaires 10 et 130 et la pièce 140 sont dans la configuration illustrée aux figures 22 à 24. En poursuivant l'entrainement de la tige 133 suivant un mouvement de translation noté F₁₀₀ sur la figure 24, le chirurgien déplace vers l'arrière, par rapport au tibia T, l'extrémité de tige 132 et, de ce fait, la pièce d'accrochage 140 à laquelle elle est fixée par l'enroulement 142. Comme représenté sur la figure 25, la pièce 140 s'engage alors à l'intérieur de l'ouverture traversante 15 en forme de fente, décrite en détail précédemment, en regard des figures 3, 6 et 7. Plus précisément, la pointe en V 141 est progressivement introduite dans l'ouverture 15, depuis la surface inférieure 13A jusqu'à la surface supérieure 13B de l'extrémité 13 du corps 12, moyennant une déformation élastique de cette pointe en V, par diminution de son angle au sommet. En pratique, on comprend que cela nécessite que la pointe en V 141 et l'ouverture 15 soient positionnées l'une par rapport à l'autre de manière à s'étendre dans un même plan géométrique : pour effectuer ce positionnement, le manche 134 porte avantageusement un moyen visuel d'indexation, non représenté, permettant au chirurgien d'indexer la position angulaire de l'ancillaire 130 par rapport à l'ancillaire 10.

L'entrainement en translation de la tige 133 est poursuivie par le chirurgien jusqu'à ce que la pointe en V 141 émerge à l'extérieur de l'ouverture 15, du côté de la surface 13B, comme représenté sur la figure 25. L'extrémité libre de la pointe en V 141 s'écarte alors automatiquement du reste de la pointe, sous l'effet de sa flexibilité par rapport au reste de la pièce 140, accrochant ainsi mécaniquement cette pièce au corps 12, en venant en prise avec la surface 13B.

Dans un quatrième temps opératoire, le chirurgien dégage l'ancillaire 130 : comme indiqué par la flèche F₁₀₁ à la figure 26, il retire la tige 133 du tunnel T₄, en la passant par l'entrée de ce tunnel. Lors du retrait de l'ancillaire 130, la pièce d'accrochage 140 se désolidarise de la tige 133, par rupture de la fixation amovible entre l'enroulement 142 et l'extrémité de tige 132, en raison de l'accrochage de la pointe en V₁₄₁ au corps 12 de l'ancillaire 10.

La suite de l'intervention chirurgicale est identique à celle décrite plus haut, en vue de tracter à l'intérieur du tunnel tibial T₄ puis de positionner la greffe G.

Par rapport aux pièces d'accrochage 40, 50, 60, 70 et 80 et à l'ancillaire 30, la pièce d'accrochage 140 et l'ancillaire 130 présentent l'avantage de simplifier encore davantage les gestes du chirurgien, tout en assurant un accrochage fiable de la pièce 140 au corps 12 de l'ancillaire 10.

Divers aménagements et variantes aux ancillaires 10, 30 et 130, ainsi qu'aux pièces d'accrochage 40, 50, 60, 70, 80 et 140 sont par ailleurs envisageables. A titre d'exemples :
- une gaine d'adaptation peut être rapportée autour du manchon 33 ou de la tige 133 lorsque le diamètre extérieur de ce dernier ou de cette dernière est inférieur au diamètre du tunnel T₄, de manière à rendre co-axiaux le manchon ou la tige et le tunnel et positionner ainsi efficacement l'extrémité 32 du manchon ou 132 de la tige par rapport à l'extrémité 13 du corps 12 de l'ancillaire de protection 10 ; une série homotétique de plusieurs gaines d'adaptation peut ainsi être prévue si des forêts 20 de plusieurs tailles sont susceptibles d'être utilisés ;
- l'ouverture dans laquelle est introduite la pièce d'accrochage pour s'accrocher au corps 12 de l'ancillaire peut n'être prévue débouchante que sur la surface inférieure 13A de l'extrémité 13, à condition que cette ouverture présente des reliefs intérieurs permettant à la pièce d'accrochage de s'y fixer mécaniquement ; en pratique, eu égard aux dimensions de l'extrémité 13, nécessairement petites pour être introduite par l'avant du genou jusqu'à la face postérieure T₃ de l'épiphyse T₂, une ouverture traversante, telle que les ouvertures 15 à 19, est plus facile à réaliser et plus efficace pour l'accrochage de la pièce liée au fil 31 ;
- une variante de réalisation, non représentée, pour la pièce d'accrochage 40, 50, 60, 70 ou 80 consiste à agencer celle-ci non pas pour l'essentiel à l'intérieur de l'extrémité 32 du manchon 33, mais à l'extérieur de cette extrémité 32, par exemple sous la forme d'un téton externe ; dans ce cas, le fil 31 n'a pas à traverser la paroi du manchon 33 mais peut s'étendre directement à l'extérieur du manchon, en courant le long de ce dernier ; cette solution est moins compacte et plus délicate à manipuler, en particulier lors de l'introduction du manchon dans le tunnel tibial T₄, mais elle permet, comme avec le sixième mode de réalisation des figures 22 à 26, d'arrimer la greffe G à l'extrémité 31A du fil dès le début de l'intervention chirurgicale ;
- sous réserve de prévoir des moyens d'indexage angulaire adéquats entre le manchon 33 et le corps 12, une seule patte flexible 41 ou 51 peut être intégrée à la pièce d'accrochage 40 ou 50 ; de même, un seul flanc cranté peut être prévu pour la pièce 70 ; et/ou
- une partie de fixation amovible à l'extrémité distale 132 de la tige 133 de l'ancillaire 130 peut, en variante non représentée, être intégrée aux différentes formes de réalisation des pièces d'accrochage 40, 50, 60, 70 et 80, afin d'utiliser ces pièces d'accrochage avec l'ancillaire 130.

## Revendications

1. Ensemble d'instrumentation chirurgicale pour reconstruire un ligament croisé postérieur d'un genou, comportant un ancillaire (10) de protection de parties molles postérieures du genou, notamment de l'artère poplitée, cet ancillaire de protection comprenant un corps rigide allongé (12), adapté pour être introduit, depuis la face antérieure du genou, dans l'espace interglénoïdien de l'épiphyse supérieure (T₂) du tibia (T) jusqu'à ce qu'une extrémité distale (13) de ce corps recouvre une zone de la face postérieure (T₃) de l'épiphyse,
**caractérisé en ce qu'**il comporte également un ancillaire (30 ; 130) de manipulation d'un fil (31) d'arrimage d'une greffe de ligament croisé postérieur (G), cet ancillaire de manipulation incluant une partie distale allongée (33, 38 ; 133), à l'extrémité distale (32 ; 132) de laquelle est agencée une pièce d'accrochage (40 ; 50 ; 60 ; 70 ; 80 ; 140) liée au fil et qui est adaptée pour être introduite, depuis la face antérieure du tibia (T), dans un tunnel (T₄) réalisé à travers le tibia et débouchant sur la face postérieure (T₃) de l'épiphyse (T₂) au niveau de la zone de cette face recouverte par l'extrémité distale (13) du corps (12) de l'ancillaire de protection (10), pour pousser vers l'arrière la pièce d'accrochage jusque dans au moins une ouverture (15 ; 16 ; 17 ; 18 ; 19) délimitée par l'extrémité distale du corps pour accrocher mécaniquement cette pièce et ce corps l'un à l'autre.

2. Ensemble suivant la revendication 1, **caractérisé en ce que** ladite partie distale allongée de l'ancillaire de manipulation (30) comprend, d'une part, un manchon (33), à l'extrémité distale (32) duquel la pièce d'accrochage (40 ; 50 ; 60 ; 70 ; 80) est agencée, notamment pour l'essentiel à l'intérieur de cette extrémité, et qui est adapté pour être introduit dans le tunnel tibial (T₄) et, d'autre part, un poussoir (37), qui est reçu de manière mobile dans le manchon et qui est adapté pour pousser vers l'arrière la pièce d'accrochage et la dégager ainsi du manchon, jusque dans la ou l'une des ouvertures (15 ; 16 ; 17 ; 18 ; 19).

3. Ensemble suivant la revendication 1, **caractérisé en ce que** ladite partie distale allongée comprend une tige (133) à l'extrémité distale (132) de laquelle est fixée de manière amovible une première partie (142) de la pièce d'accrochage (140), distincte d'une deuxième partie (141) de celle-ci conformée pour s'accrocher à l'extrémité distale (13) du corps (12) de l'ancillaire de protection (10) lorsque la pièce d'accrochage est passée à travers la ou l'une des ouvertures (15), de telle sorte que, tant que la deuxième partie n'est pas accrochée au corps, la pièce d'accrochage est reliée fixement à la tige par coopération mécanique entre la première partie et l'extrémité distale de la tige, tandis que, lorsque la deuxième partie est accrochée au corps, l'entrainement (F₁₀₁) de la tige vers l'avant rompt la fixation entre la première partie et l'extrémité distale de la tige.

4. Ensemble suivant la revendication 3, **caractérisé en ce que** la pièce d'accrochage (140) présente une structure filaire élastique, qui, au niveau de la première partie, est conformée en un enroulement (142) sensiblement centré sur l'axe longitudinal de la tige (133) lorsque cette première partie est fixée à la tige.

5. Ensemble suivant l'une des revendications 3 ou 4, **caractérisé en ce que** la pièce d'accrochage (140) présente une structure filaire élastique, qui, au niveau de la deuxième partie, est conformée en une pointe en V (141), à la fois convergente à l'opposé de la tige (133) et sensiblement centrée sur l'axe longitudinal de cette tige lorsque la première partie (142) est fixée à la tige.

6. Ensemble suivant l'une quelconque des revendications 3 à 5, **caractérisé en ce que** la pièce d'accrochage (140) présente une structure filaire élastique, qui court le long de la tige (133), notamment de manière sensiblement rectiligne, depuis la première partie (142), en direction opposée à la deuxième partie (141), jusqu'à l'extrémité proximale de la tige lorsque la première partie est fixée à la tige.

7. Ensemble suivant l'une quelconque des revendications précédentes, **caractérisé en ce que** la pièce d'accrochage (40 ; 50) comporte au moins une patte (41 ; 51) flexible de manière élastique par rapport au reste (42 ; 52) de la pièce d'accrochage, la ou chaque patte étant apte à passer d'une première configuration, dans laquelle la patte est contrainte dans une position déformée par rapport au reste de la pièce d'accrochage pour passer à travers la ou l'une des ouvertures (15 ; 16) de l'extrémité (13) du corps (12), à une seconde configuration, dans laquelle la patte est rappelée de manière élastique depuis sa position déformée pour accrocher le corps.

8. Ensemble suivant la revendication 7, **caractérisé en ce que** la ou chaque patte (41 ; 51) comporte une extrémité libre (43 ; 53) formant un bord saillant par rapport au reste de la patte et adaptée pour venir en prise avec une zone de débouché vers l'arrière (15A ; 16B) de l'ouverture correspondante (15 ; 16) lorsque la patte est dans sa seconde configuration.

9. Ensemble suivant l'une des revendications 7 ou 8, **caractérisé en ce que** la pièce d'accrochage (40) inclut deux pattes flexibles (41) qui sont reliées l'une à l'autre, à une de leurs extrémités, par un pont (42) de manière que la pièce d'accrochage présente une forme globale de fer à cheval, ces deux pattes étant rapprochées l'une de l'autre dans leur première configuration et s'écartant automatiquement l'une de l'autre sous l'effet de leur flexibilité lorsqu'elles passent de leur première à leur seconde configuration.

10. Ensemble suivant l'une des revendications 7 ou 8, **caractérisé en ce que** la pièce d'accrochage (50) inclut deux pattes flexibles (51) qui sont reliées l'une à l'autre, à une de leurs extrémités, par un pont (52) de manière que la pièce d'accrochage présente une forme globale de U, les deux pattes étant écartées l'une de l'autre dans leur première configuration et se rapprochant automatiquement l'une de l'autre sous l'effet de leur flexibilité lorsqu'elles passent de leur première à leur seconde configuration.

11. Ensemble suivant la revendication 10, **caractérisé en ce que** les deux pattes (51) sont respectivement associées à deux ouvertures (16) délimitées par des flancs opposés de l'extrémité distale (13) du corps (12).

12. Ensemble suivant la revendication 11, **caractérisé en ce que** les deux ouvertures (16) définissent respectivement des surfaces de rampe (16A) pour des extrémités libres (53) des pattes (51) de telle sorte que ces surfaces de rampe écartent les pattes l'une de l'autre lorsque la pièce d'accrochage (50) est poussée dans ces ouvertures par l'ancillaire de manipulation (30).

13. Ensemble suivant l'une quelconque des revendications 1 à 6, **caractérisé en ce que** la pièce d'accrochage (60) présente une forme allongée de section transversale sensiblement constante suivant sa longueur, tandis que l'ouverture (17) présente une forme allongée dont la section transversale est sensiblement complémentaire de celle de la pièce, de telle sorte que la pièce d'accrochage est apte à être totalement passée à travers l'ouverture lorsqu'elle est poussée par l'ancillaire de manipulation (30) puis à basculer d'elle-même contre la surface (13B), tournée à l'opposé de l'ancillaire de manipulation (30), de l'extrémité distale (13) du corps (12) pour accrocher ce corps.

14. Ensemble suivant l'une quelconque des revendications 1 à 6, **caractérisé en ce que** la pièce d'accrochage (70) présente une forme allongée munie, sur au moins un de ses flancs, de crans saillants (75) répartis suivant sa longueur, tandis que l'ouverture (18) de l'extrémité (13) du corps (12) est munie d'au moins une lamelle (18A) apte, d'une part, à être déformée par les crans (75) pour laisser passer la pièce vers l'arrière à travers l'ouverture lorsque la pièce d'accrochage est poussée par l'ancillaire de manipulation (30) et, d'autre part, à bloquer la pièce d'accrochage, par coopération avec l'un des crans, en sens inverse pour accrocher le corps.

15. Ensemble suivant l'une quelconque des revendications 1 à 6, **caractérisé en ce que** la pièce d'accrochage (80) comporte un corps cylindrique (81) muni, à son extrémité distale, d'une barrette transversale (83), tandis que l'ouverture (19) de l'extrémité distale (13) du corps (12) présente une section transversale sensiblement complémentaire de celle de la barrette, de telle sorte que la pièce d'accrochage est apte à être partiellement passée à travers l'ouverture puis entraînée en rotation sur elle-même autour de l'axe longitudinal central de son corps de manière que la barrette (83) s'étende en travers du débouché (19A) de l'ouverture sur la surface (13B), tournée à l'opposé de l'ancillaire de manipulation (30), de l'extrémité distale du corps pour accrocher le corps.
